## Europäisches Patentamt

## European Patent Office

## Office européen des brevets

(19)

(11) Veröffentlichungsnummer: **0 129 534**
**B1**

(12)

# EUROPÄISCHE PATENTSCHRIFT

(45) Veröffentlichungstag der Patentschrift:
23.08.89

(51) Int. Cl.⁴: **C 08 B 37/10, A 61 K 31/725**

(21) Anmeldenummer: **84890084.1**

(22) Anmeldetag: **10.05.84**

(54) Verfahren zur Herstellung eines Antithrombin III-Heparin- bzw. Antithrombin III-Heparinoid-Konzentrates.

(30) Priorität: **20.05.83 AT 1859/83**

(43) Veröffentlichungstag der Anmeldung:
**27.12.84 Patentblatt 84/52**

(45) Bekanntmachung des Hinweises auf die Patenterteilung:
**23.08.89 Patentblatt 89/34**

(84) Benannte Vertragsstaaten:
**AT BE CH DE FR GB IT LI NL SE**

(56) Entgegenhaltungen:
**DD-A-148 297**
**US-A-3 099 600**
**US-A-3 842 061**
**US-A-4 119 774**
**US-A-4 175 182**

**CHEMICAL ABSTRACTS, Band 92, Nr. 23, 9. Juni 1980, Seite 31, Nr. 191134d, Columbus, Ohio, US; T. KITANI u.a.: "Effect of protamine on heparin-antithrombin III complexes. In vitro studies" & THROMB. RES. 1980, 17(3-4), 367-74**

(73) Patentinhaber: **IMMUNO Aktiengesellschaft für chemisch- medizinische Produkte, Industriestrasse 72, A-1220 Wien (AT)**

(72) Erfinder: **Eibl, Johann, Dr., Gustav Tschermakgasse 2, A-1180 Wien (AT)**
Erfinder: **Hetzl, Ernst, Dr. Dipl.Ing., Bergheidengasse 8/1/9, A-1130 Wien (AT)**
Erfinder: **Linnau, Yendra, Dr., Lavendlweg 24, A-1220 Wien (AT)**

(74) Vertreter: **Wolfram, Gustav, Dipl.- Ing., Schwindgasse 7 P.O. Box 205, A-1041 Wien (AT)**

## Beschreibung

Die Erfindung betrifft ein Verfahren zur Herstellung eines Antithrombin III-Heparin- bzw. Antithrombin III-Heparinoid-Konzentrates.

Es ist bekannt, daß Antithrombin III zusammen mit Heparin bzw. Heparinoiden einen Komplex bildet, der pharmazeutisch wertvolle Eigenschaften aufweist; insbesondere werden durch die Gegenwart des Komplexes bei der Herstellung und Lagerung von Konzentraten hochwirksamer Gerinnungsfaktoren und anderer Plasmaproteine mit biologischer Wirksamkeit enzymatisch bedingte Proteinveränderungen, die unerwünschte Nebenreaktionen am Patienten zur Folge haben können, unterdrückt. Auch Konzentrate von Antithrombin III haben eine ähnliche Wirkung. Weitere pharmazeutisch wertvolle Eigenschaften dieser Produkte sind ihre Wirksamkeit gegenüber Thromboembolismus bei angeborenem Mangel an Antithrombin III sowie bei Risikopatienten, bei denen unter Heparintherapie Antithrombin III-Abfall eintritt.

In der AT-B-359 646 ist die Bildung eines Antithrombin III-Heparin-Komplexes beschrieben, indem zu menschlichem Citratplasma oder zu gereinigtem Antithrombin III Heparin zugefügt wird. Die Mischung wird Faktor VIIIhältigen Fraktionen zu Stabilisierungszwecken zugefügt. Eine Isolierung des Komplexes erfolgt hierbei nicht.

In der veröffentlichten europäischen Patentanmeldung 0 048 898 ist die Herstellung eines Antithrombin III-Heparin-Komplexes beschrieben, indem vorgereinigtes Antithrombin III und Heparin an immobilisiertem Lectin adsorbiert, das überschüssige Heparin durch Waschen entfernt und der Komplex mit Kohlehydratlösungen eluiert wird. Hierbei ergibt sich ein Problem mit der Leakage des Liganden, da die verwendeten Lectine mitogene Wirkung haben. Daher ist die therapeutische Anwendung eines so hergestellten Produktes bedenklich.

In der Literaturstelle "The Chemistry and Physiology of the Human Plasma Proteins" von R.D. Rosenberg, Ed. H. Bing, Pergamon Press, 1979, Seite 353, beschreibt Rosenberg die Herstellung des Komplexes aus gereinigtem Antithrombin III und Heparin, wobei der Komplex von überschüssigem Heparin durch Gelfiltration abgeschieden und isoliert wird. Weiters kann nach Rosenberg der Komplex durch Gelfiltration in Anwesenheit von 3 M NaCl-Lösung in seine Bestandteile zerlegt werden.

So weit bisher Antithrombin III-Heparin-Komplexe als solche, d.h. als Reinsubstanzen, hergestellt worden sind, erfolgte dies hauptsächlich für analytische Zwecke. Eine Gelfiltration bzw. eine Affinitätschromatographie mit toxischen Liganden, wie Lectinen, sind für eine Produktion therapeutisch verwendbarer Produkte keine geeigneten Methoden.

Die Erfindung bezweckt die Vermeidung dieser Nachteile und Schwierigkeiten und stellt sich die Aufgabe, Antithrombin III-Heparin- bzw. Antithrombin III-Heparinoid-Komplexkonzentrate im Produktionsmaßstab herstellen zu können, d.h. um Produkte zu erhalten, die für die Anwendung als Therapeutika geeignet sind.

Die Erfindung geht aus von einem Verfahren zur Herstellung eines Antithrombin III-Heparin- bzw. Antithrombin III-Heparinoid-Konzentrates, wobei Humanplasma oder Antithrombin III-hältige Plasmafraktionen unter Bildung eines Antithrombin III-Heparin- bzw. Antithrombin III-Heparinoid-Komplexes mit Heparin bzw. Heparinoid versetzt werden, und ist dadurch gekennzeichnet, daß der gebildete Antithrombin III-Heparin- bzw. Antithrombin III-Heparinoid-Komplex an einem Anionenaustauscher adsorbiert, das Adsorbat durch eine Salzlösung eluiert, der in dem Eluat enthaltene Antithrombin III-Heparin- bzw. Antithrombin III-Heparinoid-Komplex von Salzen und unerwünschten Proteinen getrennt und das erhaltene Produkt durch Lyophilisieren in haltbare Form gebracht wird.

Im Gegensatz zu den einleitend erwähnten Herstellungsmethoden sind die erfindungsgemäßen Maßnahmen äußerst einfach und mit guter Ausbeute durchzuführen. Eine teilweise Denaturierung des Antithrombin III findet beim erfindungsgemäßen Verfahren nicht statt. Man erreicht eine sehr gute Heparinbindungsfähigkeit des Antithrombin III, und das Heparin hat hohe Affinität zu Antithrombin III, obwohl keiner der beiden Partner vorher gereinigt werden muß. Als Anionenaustauscher können beispielsweise solche auf Basis von quervernetzten Dextrangelen (z. B. DEAE-Sephadex, QAE-Sephadex) oder DEAE-Cellulose eingesetzt werden. Zweckmäßig wird das Adsorbat vor der Eluierung gewaschen.

Gemäß einer vorteilhaften Ausführungsform wird das Eluat mit Proteinfällungsmitteln, wie Ammoniumsulfat, behandelt, in einer Konzentration, die ausreicht, um den Antithrombin III-Heparin- bzw. Antithrombin III-Heparinoid-Komplex zu fällen, worauf der Niederschlag gelöst, die Lösung hitzeinaktiviert und das Produkt sodann lyophilisiert wird. Die Hitzebehandlung zum Inaktivieren eventuell vorhandener Infektionserreger kann durch Erwärmen auf 60°C während einer Dauer von 10 Stunden vorgenommen werden.

Bei dieser Arbeitsweise kann zusätzlich nach dem Hitze-Inaktivierungsschritt die den Antithrombin III-Heparin- bzw. Antithrombin III-Heparinoid-Komplex enthaltende Lösung mit einem Proteinfällungsmittel behandelt werden, u. zw. in einer Konzentration, die ausreicht, um unerwünschte Proteine zu entfernen, jedoch den Komplex selbst in Lösung läßt.

Das erfindungsgemäße Verfahren wird durch folgende Beispiele näher erläutert:

**Beispiel 1:**

Herstellung des Antithrombin III-Heparin-Komplexes aus Plasma

Zu 100 l Plasma wurden nach der Entfernung des Kryopräzipitates 8.10[6] E Heparin zugesetzt und es wurde 30 min bei +4°C gerührt. Nach dem Einrühren von 100 g DEAE-Sephadex A 50 wurde weitere 2 Stunden bei +4°C gerührt. Das beladene Gel wurde mittels Büchnertrichter abgetrennt und zur Entfernung von Begleitproteinen mit 2 l einer Phosphat-Citrat-gepufferten, isotonen Kochsalzlösung eines pH-Wertes von 7,5 gewaschen. Das beladene, gewaschene Gel wurde in 2,2 l des oben genannten Puffers suspendiert, und durch Zugabe von festem NaCl wurde eine Leitfähigkeit von 42 mS/cm eingestellt. Nach einstündigem Rühren bei +4°C wurde mittels Büchnertrichter abgetrennt und mit 0,7 l einer Phosphat- und Citrat-gepufferten Kochsalzlösung mit einer Leitfähigkeit von 42 mS/cm nachgewaschen.

Aus den vereinigten Eluaten wurde durch Zugabe von 1,4 kg Ammoniumsulfat und Einstellen des pH-Wertes auf 5,5 der Antithrombin III-Heparin-Komplex ausgefällt. Die hierbei angewendete Ammoniumsulfatkonzentration von 430 g/l entspricht einer 80 -%-igen Sättigung der Lösung. Nach einstündigem Rühren bei +4°C wurde der Niederschlag durch Filtration abgetrennt und gemeinsam mit 13,5 g NaCl und 221 g Na$_3$Citrat · 2 H$_2$O in 1,5 l destilliertem Wasser gelöst.

Der pH-Wert wurde auf 7,5 eingestellt und zur Inaktivierung von eventuell vorhandenen Hepatisviren wurde 10 Stunden auf 60°C erhitzt.

Das pasteurisierte Produkt wurde gegen 50 l isotone Kochsalzlösung dialysiert und zur weiteren Reinigung wurden bei einem pH-Wert von 7,0 303 g Ammoniumsulfat je 1 l eingerührt. Die hierbei angewendete Ammoniumsulfatkonzentration von 270 g/l entspricht einer 50 -%-igen Sättigung der Lösung. Nach 45 min Rührdauer bei +4°C wurde der Niederschlag durch Zentrifugieren abgetrennt und verworfen.

Der Oberstand wurde gegen 100 l einer Citrat-gepufferten, isotonen Kochsalzlösung dialysiert und durch Ultrafiltration auf einen Antithrombin III-Gehalt von 50 E/ml eingeengt.

Nach der Dialyse gegen eine Citrat-gepufferte isotone Kochsalzlösung wurde sterilfiltriert, in Flaschen abgefüllt und gefriergetrocknet.

Die enzymatischen Aktivitäten von Antithrombin III und Heparin wurden in folgender Weise getestet:

Enzymatische Aktivität von Antithrombin III:
Die Aktivität wurde auf eine Standardpräparation bezogen, die gegen Antithrombin III Plasma Human (1. Int. R.P. 72/1) geeicht worden war. Mit dieser Standardpräparation wurde eine Eichkurve ermittelt, auf der die zu testenden Proben abgelesen wurden.

Die Verdünnung der Proben und des Standards auf Aktivitäten zwischen 0,0125 und 0,0625 E/ml erfolgte mit einer Pufferlösung folgender Zusammensetzung:

| 3,03 | g Tris | ) |
|------|--------|---|
| 10,8 | g NaCl | ) |
| 1,4 | g Na-EDTA | ) je 1 l, PH 8,4 |
| 3000 | E Heparin | ) |

Pipettierschema:

a) 0,1 ml Probe wurde auf 37°C erwärmt
b) 0,1 ml Thrombin (12 IE/ml) wurden zugesetzt und es wurde 3 min bei 37°C inkubiert
c) 0,1 ml einer 1,2 mM Lösung von TH-1 (2 AcOH.H-D-CHG-Ala-Arg-pNA) wurden zugesetzt
d) nach genau 1 min bei 37°C wurde die Reaktion durch Zugabe von 0,1 ml 20 % Essigsäure gestoppt
e) die Extinktion bei 405 nm wurde gemessen

Enzymatische Aktivität von Heparin:
Die Aktivität wurde auf eine Standardpräparation bezogen, die gegen den 3. Int. Stand. Heparin 65/69 geeicht worden war. Mit dieser Standardpräparation wurde eine Eichkurve ermittelt, auf der die zu testenden Proben abgelesen wurden.

Die Verdünnung der Proben und des Standards auf Aktivitäten von 0,001 bis 0,5 IE/ml erfolgte mit einer Pufferlösung folgender Zusammensetzung:

| 6,06 | g Tris | ) |
|------|--------|---|
| 18,12 | g NaCl | ) je 1 l, pH 8,3 |
| 2,79 | g Na-EDTA | ) |

Pipettierschema:

a) 0,2 ml Probe und 0,2 ml heparinfreies Antithrombin III (3 E/ml) wurden 20 min bei Raumtemperatur und 3 bis 5 min bei 37°C inkubiert
b) 0,2 ml Thrombin (20 IE/ml) wurden zugesetzt und es wurde 1,5 min bei 37°C inkubiert
c) 0,2 ml einer 1,2 mM Lösung von TH-1 wurden zugesetzt
d) nach genau 1 min bei 37°C wurde die Reaktion durch Zugabe von 0,2 ml 50 % Eisessig gestoppt
e) die Extinktion bei 405 nm wurde gemessen Dabei ergab sich für das nach dem Beispiel 1 erhaltene Endprodukt ein Heparingehalt von 5 bis 7 Heparineinheiten pro Einheit Antithrombin III.

**Beispiel 2:**

Herstellung eines Antithrombin III-Heparinoid-Komplexes:

Zu 1 l Plasma wurden 3 g des Heparinoides Pentosanpolysulfat-Natriumsalz (Polyanion SP 54) zugesetzt, worauf 30 min bei +4°C gerührt wurde. Nach dem Einrühren von 2,5 g DEAE-Sephadex A 50 wurde während weiterer 2 Stunden bei +4°C gerührt. Das beladene Gel wurde mittels Büchnertrichter abgetrennt und zur Entfernung von Begleitproteinen zweimal mit je 200 ml einer Phosphat- und Citrat-gepufferten, isotonen Kochsalzlösung gewaschen. Das beladene, gewaschene Gel wurde in 100 ml des oben genannten Puffers suspendiert, und durch Zugabe von festem Kochsalz eine Leitfähigkeit von 60 mS/cm eingestellt. Nach einständigem Rühren bei +4°C wurde mittels Büchnertrichter abgetrennt und der Antithrombin III-SP 54-Komplex gewonnen.

Die weitere Verarbeitung des Eluates zum Endprodukt erfolgte wie in Beispiel 1 beschrieben.

Die enzymatische Analyse lieferte einen Heparinoid-Gehalt, der einer Aktivität von 2 bis 3 E Heparin je 1 E Antithrombin III entspricht.

## Patentansprüche

1. Verfahren zur Herstellung eines Antithrombin III-Heparin- bzw. Antithrombin III-Heparinoid-Konzentrates, wobei Humanplasma oder Antithrombin III-hältige Plasmafraktionen unter Bildung eines Antithrombin III-Heparin- bzw. Antithrombin III-Heparinoid-Komplexes mit Heparin bzw. Heparinoid versetzt werden, dadurch gekennzeichnet, daß der gebildete Antithrombin III-Heparin- bzw. Antithrombin III-Heparinoid-Komplex an einem Anionenaustauscher adsorbiert, das Adsorbat durch eine Salzlösung eluiert, der in dem Eluat enthaltene Antithrombin III-Heparin- bzw. Antithrombin III-Heparinoid-Komplex von Salzen und unerwünschten Proteinen getrennt und das erhaltene Produkt durch Lyophilisieren in haltbare Form gebracht wird.

2. Verfahren nach Anspruch 1, dadurch gekennzeichnet, daß das Adsorbat vor der Eluierung gewaschen wird.

3. Verfahren nach Anspruch 1 oder 2, dadurch gekennzeichnet, daß das Eluat mit Proteinfällungsmitteln, wie Ammoniumsulfat, behandelt wird, in einer Konzentration, die ausreicht, um den Antithrombin III-Heparin- bzw. Antithrombin III-Heparinoid-Komplex zu fällen, worauf der Niederschlag gelöst, die Lösung hitze-inaktiviert und sodann lyophilisiert wird.

4. Verfahren nach Anspruch 3, dadurch gekennzeichnet, daß nach der Hitze-Inaktivierung die den Antithrombin III-Heparin- bzw. Antithrombin III-Heparinoid-Komplex enthaltende Lösung mit einem Proteinfällungsmittel, wie Ammoniumsulfat, behandelt wird, in einer Konzentration, die ausreicht, um unerwünschte Proteine zu entfernen, jedoch den Komplex selbst in Lösung läßt.

## Claims

1. Method of producing an antithrombin III-heparin concentrate or an antithrombin III-heparinoid concentrate, wherein heparin or heparinoid is added to human plasma or to antithrombin III-containing plasma fractions under formation of an antithrombin III-heparin complex or an antithrombin III-heparinoid complex, characterised in that, the antithrombin III-heparin complex or the antithrombin III-heparinoid complex formed is adsorbed on an anion exchanger, the adsorbate is eluted, with a salt solution, the antithrombin III-heparin or antithrombin III-heparinoid complex contained in the eluate is separated from salts and undesired proteins, and the resulting product is brought into stable form by lyophilisation.

2. Method according to claim 1, characterised in that the adsorbate is washed before elution.

3. Method according to claim 1 or 2, characterised in that the eluate is treated with protein precipitating agents, such as ammonium sulfate, in a concentration sufficing to precipitate the antithrombin III-heparin complex or the antithrombin III-heparinoid complex, whereupon the precipitate is dissolved, the solution is thermally inactivated and subsequently lyophilised.

4. Method according to claim 3, characterised in that after thermal inactivation the solution containing the antithrombin III-heparin complex or the antithrombin III-heparinoid complex is treated with a protein precipitating agent, such as ammonium sulfate, in a concentration sufficing to remove undesired proteins, but leaving the complex itself in solution.

## Revendications

1. Procédé pour la fabrication d'un concentré antithrombine III-héparine ou antithrombine III-héparinoïde, dans lequel on ajoute de l'héparine ou de l'héparinoïde à du plasma humain ou à des fractions de plasma contenant de l'antithrombine III avec formation d'un complexe antithrombine III-héparine ou antithrombine III-héparinoïde, caractérisé en ce que le complexe antithrombine III-héparine ou antithrombine III-héparinoïde formé est adsorbé sur un échangeur d'anions, l'adsorbat est élué par une solution saline, le complexe antithrombine III-héparine ou antithrombine III-héparinoïde contenu dans l'éluat est séparé des sels et des protéines indésirables et le produit obtenu est mis sous une forme stable par lyophilisation.

2. Procédé selon la revendication 1, caractérisé en ce que l'adsorbat est lavé avant l'élution.

3. Procédé selon la revendication 1 ou 2, caractérisé en ce que l'éluat est traité par des agents de précipitation des protéines, tels que le sulfate d'ammonium, à une concentration qui est suffisante pour précipiter le complexe antithrombine III-héparine ou antithrombine III-héparinoïde, après quoi le précipité est dissous, la solution est inactivée par la chaleur et ensuite lyophilisée.

4. Procédé selon la revendication 3, caractérisé en ce que la solution contenant le complexe antithrombine III-héparine ou antithrombine III-hé-

héparinoïde est traitée après l'inactivation par la chaleur avec un agent de précipitation des protéines, tel que le sulfate d'ammonium, à une concentration qui est suffisante pour séparer les protéines indésirables, mais qui laisse le complexe lui même en solution.